# EUROPEAN PATENT APPLICATION

(11) **EP 4 201 396 A1**
(43) Date of publication of application: **28.06.2023**
(21) Application number: 21892083.3
(22) Date of filing: 03.08.2021
(51) Int. Cl.: A61K 8/9789, A61K 8/06, A61Q 19/02

(54) **WHITENING COSMETIC COMPOSITION COMPRISING EXOSOMES DERIVED FROM ROSE STEM CELLS AS ACTIVE INGREDIENT**

(30) Priority: 16.11.2020 KR 20200152457
(71) Applicant: Exocobio Inc., Seoul 08594 (KR)
(72) Inventor: CHO, Byong Seung, Gunpo-si Gyeonggi-do 15835 (KR)
(74) Representative: Isarpatent
(86) International application number: PCT/KR2021/010135
(87) International publication number: WO 2022/102913

(57) **Abstract**

The present invention provides a cosmetic composition for whitening a skin containing rose stem cell-derived exosomes as an active ingredient. The cosmetic composition of the present invention has excellent effects on skin whitening, skin beauty and the like.

## Description

### TECHNICAL FIELD

The present invention relates to a cosmetic composition for whitening a skin comprising rose stem cell-derived exosomes as an active ingredient.

### BACKGROUND ART

Human skin color is determined by the concentration and distribution of melanin in the skin. Melanin is synthesized through a non-enzymatic oxidation reaction after conversion from tyrosine to DOPA and dopaquinone by tyrosinase. When melanin is excessively produced, pigmentation occurs, and then melasma, spots and freckles appear on face, neck, arms and the like, which are not good for appearance. Although skin whitening agents are being developed to improve melasma, freckles and dark skin tone, it is difficult to develop skin whitening agents that have an excellent whitening effect and have no side effect. For example, a skin whitening agent that selectively attacks melanocytes that produce melanin has an excellent whitening effect, but has a side effect of skin toxicity.

Recently, there have been reports that cell secretomes contain various bioactive molecules that regulate cellular behaviors. In particular, cell secretomes contain 'exosome' or 'extracellular vesicle' that has intercellular signaling functions, and thus studies on the components and functions thereof have been actively conducted.

Cells shed various membraneous vesicles to their extracellular environment, and these released vesicles are usually called extracellular vesicles (EVs). The EV is also called cell membrane-derived vesicle, ectosome, shedding vesicle, microparticle, exosome, etc., and is also used discriminately from exosome in some cases.

Exosome is a vesicle of tens to hundreds of nanometers in size, which comprises a phospholipid bilayer membrane having the same structure as that of the cell membrane. This exosome contains proteins, nucleic acids (mRNA, miRNA, etc.) and the like which are called exosome cargo. It is known that exosome cargo includes a wide range of signaling factors, and these signaling factors are specific for cell types and regulated differently depending on secretory cells' environment. It is known that exosome is an intercellular signaling mediator secreted by cells, and various cellular signals transmitted through it regulate cellular behaviors, including the activation, growth, migration, differentiation, dedifferentiation, apoptosis, and necrosis of target cells. Exosome contains specific genetic materials and bioactive factors depending on the nature and state of cells from which the exosome was derived. Exosome derived from proliferating stem cells regulates cell behaviors such as cell migration, proliferation and differentiation, and recapitulates the characteristics of stem cells involved in tissue regeneration (Nature Review Immunology 2002 (2) 569-579).

That is, exosomes called "avatars" of cells contain bioactive factors such as growth factors, similar to cells, and serve as carriers that transmit bioactive factors between cells, that is, serve to mediate cell-to-cell communication. Exosomes are known to be released not only from animal cells such as stem cells, immune cells, fibroblasts and cancer cells, but also from cells of various organisms such as plants, bacteria, fungi, and algae. For example, exosomes may be isolated from conditioned media of plant stem cells, as well as conditioned media of cancer cells, immune cells, mesenchymal stem cells, and the like.

However, studies on the isolation, purification, and characterization of exosomes derived from plant stem cells remain insufficient. Therefore, more detailed characterization and functional studies of exosomes derived from plant stem cells are required.

Various varieties of roses are cultivated in a wide range of areas such as the cold, subarctic, temperate, and subtropical zones of the northern hemisphere, and extract thereof are used in perfumes and cosmetics. However, technologies to date are at a level where extracts obtained by drying rose petals and subjecting the dried petals to hot-water extraction or solvent extraction are used for perfumes and air fresheners. In addition, conditioned media of rose callus contain growth regulators or a callus inducing substance, and thus are hardly regarded as natural cosmetic ingredients, and the growth regulators contained in the cultures may cause side effects such as skin troubles. Rose extract also contains impurities in the process of hot-water extraction or solvent extraction, and thus it frequently causes skin troubles or allergies to a person with sensitive skin.

The present inventor has conducted extensive studies on the isolation, purification, and characterization of rose stem cell-derived exosomes, and as a result, has found that exosomes derived from rose stem cells have a skin whitening effect, and has developed a cosmetic composition for whitening a skin comprising exosomes derived from rose stem cells as an active ingredient.

Meanwhile, it is to be understood that the matters described as the background art are intended merely to aid in the understanding of the background of the present invention and are not admitted as prior art against the present invention.

### SUMMARY OF INVENTION

An object of the present invention is to provide a cosmetic composition for whitening a skin comprising rose stem cell-derived exosomes as an active ingredient.

However, the objects of the present invention as described above are illustrative and the scope of the present invention is not limited thereby. In addition, other objects and advantages of the present invention will be more apparent from the following description, the appended claims and the accompanying drawings.

### DETAILED DESCRIPTION OF INVENTION

The present invention provides a cosmetic composition for whitening a skin containing exosomes derived from rose stem cells (rose stem cell-derived exosomes) as an active ingredient.

As used herein, the term "rose (Rosa spp.)" refers to plants belonging to the genus Rosa, in the family Rosaceae, the order Rosales and the class Dicotyledoneae, and includes all of wild species and cultivated garden species.

As used herein, the term "exosomes" refers to nano-sized vesicles secreted or released from plant cells into extracellular spaces and having a membrane structure, and is also referred to as exosome-like vesicles or exosome-like particles.

As used herein, the term "whitening" includes increasing the brightness of the skin whose brightness has decreased due to an excess of pigments such as melanin, or maintaining the brightness of the skin at a certain level.

In the cosmetic composition according to one embodiment of the present invention, the rose stem cells may be obtained by inducing a callus derived from rose embryos or leaves and then culturing cells of the callus.

The cosmetic composition according to one embodiment of the present invention may be, for example, a cream or a lotion.

Meanwhile, the cosmetic composition according to one embodiment of the present invention may suitably contain components which are generally used in cosmetic products, for example, moisturizers, antioxidants, oily components, UV absorbers, emulsifiers, surfactants, thickeners, alcohols, powder components, colorants, aqueous components, water, and various skin nutrients, etc., as needed, within the range that does not impair the effect of the present invention.

Furthermore, the cosmetic composition according to one embodiment of the present invention may include, in addition to the rose stem cell-derived exosomes, a skin whitening agent, an agent for improving skin condition and/or a moisturizer, which have been used in the prior art, within the range that does not impair the effects (e.g., skin whitening, skin beauty, etc.). For example, the rose stem cell-derived exosomes of the present invention may be contained in or mixed with at least one of hydrogel, hyaluronic acid, salt of hyaluronic acid (e.g., sodium hyaluronate, etc.), or hyaluronate gel. In the cosmetic composition according to one embodiment of the present invention, the kind of hydrogel is not particularly limited, but the hydrogel may be preferably obtained by dispersing a gelled polymer in a polyhydric alcohol. The gelled polymer may be at least one selected from the group consisting of pluronic, purified agar, agarose, gellan gum, alginic acid, carrageenan, cassia gum, xanthan gum, galactomannan, glucomannan, pectin, cellulose, guar gum, and locust bean gum, and the polyhydric alcohol may be at least one selected from the group consisting of ethylene glycol, propylene glycol, 1,3-butylene glycol, isobutylene glycol, dipropylene glycol, sorbitol, xylitol, and glycerin.

The cosmetic composition according to one embodiment of the present invention may be used in various forms, for example, a patch, a mask pack, a mask sheet, a cream, a tonic, an ointment, a suspension, an emulsion, a paste, a lotion, a gel, an oil, a pack, a spray, an aerosol, a mist, a foundation, a powder and an oilpaper. For example, the cosmetic composition may be applied to or soaked in at least one surface of a patch, a mask pack or a mask sheet.

The cosmetic composition is used for the purpose of skin whitening, skin beauty and the like, and the cosmetic composition may be prepared as any formulation which is generally prepared in the art. For example, it may be formulated as a patch, a mask pack, a mask sheet, a skin softener, a nutrition, an astringent lotion, a nourishing cream, a massage cream, an eye cream, a cleansing cream, an essence, an eye essence, a cleansing lotion, a cleansing foam, a cleansing water, a sunscreen, a lipstick, a soap, a shampoo, a surfactant-containing cleanser, a bath preparation, a body lotion, a body cream, a body oil, a body essence, a body cleanser, a hairdye, a hair tonic, etc., but is not limited thereto.

The cosmetic composition according to one embodiment of the present invention contains components which are commonly used in cosmetic products. For example, the cosmetic composition may contain conventional adjuvants and carriers, such as antioxidants, stabilizers, solubilizers, vitamins, pigments, and fragrances. In addition, other components in each formulation for the cosmetic composition may be suitably selected without difficulty by those skilled in the art depending on the type or intended use of the cosmetic composition.

Another embodiment of the present invention provides a cosmetic method for whitening a skin, using the cosmetic composition.

The cosmetic method according to one embodiment of the present invention includes: (a) applying the cosmetic composition directly to a mammalian skin; or (b) contacting or attaching a patch, a mask pack or a mask sheet, which has the cosmetic composition applied thereto or soaked therein, to the mammalian skin; or sequentially performing (a) and (b). In step (a), the cosmetic composition may be a lotion or a cream.

Alternatively, the cosmetic method according to one embodiment of the present invention may further comprise step (c) removing the patch, the mask pack or the mask sheet from the mammalian skin after step (b), and applying the cosmetic composition to the mammalian skin. In step (c), the cosmetic composition may be a lotion or a cream.

In the cosmetic method according to one embodiment of the present invention, the mammal may be a human, a dog, a cat, a rodent, a horse, a cattle, a monkey, or a pig.

### ADVANTAGEOUS EFFECTS

The cosmetic composition for whitening a skin containing rose stem cell-derived exosomes as an active ingredient according to the present invention has advantages that it is less likely to contain impurities such as growth regulators than conventional rose filtrates, rose extracts and conditioned media of rose callus, and is less cytotoxic than filtrates of conditioned media of rose stem cells and rose extracts. The cosmetic composition of the present invention has excellent effects on skin whitening, skin beauty and the like.

It should be understood that the scope of the present invention is not limited to the aforementioned effects.

### BRIEF DESCRIPTION OF DRAWINGS

FIG. 1A is a graph showing the results of NTA analysis of rose stem cell-derived exosomes (100 kDa exosomes) obtained according to Example 3.
FIG. 1B is a graph showing the results of NTA analysis of rose stem cell-derived exosomes (500 kDa exosomes) obtained according to Example 3.
FIG. 1C is a graph showing the results of NTA analysis of filtered conditioned media of rose stem cells (CM) before performing TFF in Example 3.
FIG. 2 is a graph showing that the amount of melanin production is remarkably decreased when melanoma cells are treated with rose stem cell-derived exosomes of the present invention compared to when they are treated with filtrates of conditioned media of rose stem cells.
FIG. 3 is a graph showing that the cell viability, when melanoma cells were treated with rose stem cell-derived exosomes of the present invention and cultured, was higher than that when melanoma cells were treated with filtrates of conditioned media of rose stem cells and cultured, and thus the rose stem cell-derived exosomes are less cytotoxic than the filtrates of conditioned media of rose stem cells.

### EXAMPLES

Hereinafter, the present invention will be described in more detail with reference to the following examples. However, the following examples are only to illustrate the present invention and are not intended to limit or restrict the scope of the present invention. Those that can be easily inferred by those skilled in the art from the detailed description and examples of the present invention are interpreted as falling within the scope of the present invention. References referred to in the present invention are incorporated herein by reference.

Throughout the present specification, it is to be understood that, when any part is referred to as "comprising" any component, it does not exclude other components, but may further include other components, unless otherwise specified.

### Example 1: Cell Culture

B16F10 cells, which are mouse melanoma (mus musculus melanoma) cells, purchased from ATCC were subcultured in phenol red-free DMEM (purchased from ThermoFisher Scientific) medium containing 10% fetal bovine serum (FBS; purchased from ThermoFisher Scientific), 1% antibiotics-antimycotics (purchased from ThermoFisher Scientific), L-glutamine (purchased from ThermoFisher Scientific) and sodium pyruvate (purchased from ThermoFisher Scientific) at 37°C under 5% CO₂.

### Example 2: Preparation of Rose Stem Cells

According to preparation and culture methods of plant stem cells known in the art, calluses were induced from rose embryos and/or leaves, and cells of the induced callus were cultured. In addition, a callus having a good growth state was selected and cultured in large amounts, thereby preparing conditioned media of rose stem cells.

### Example 3: Preparation of Rose Stem Cell-Derived Exosomes

The conditioned media of rose stem cells (conditioned media of rose callus) prepared as described in Example 2 were purchased from BIO-FD&C Co., Ltd. (located in Incheon, Korea and supplying conditioned media of Damask Rose stem cells). The conditioned media of rose stem cells were filtered through a 0.22 µm filter to remove impurities such as cell debris, waste products and large particles. Rose stem cell-derived exosomes were isolated from the filtered conditioned media by tangential flow filtration (TFF) method. For isolating rose stem cell-derived exosomes using the TFF method, a MWCO 100 kDa filter for the TFF or a 500 kDa filter for the TFF was used. Hereinafter, rose stem cell-derived exosomes isolated using the 100 kDa filter are referred to as "100 kDa exosomes", and rose stem cell-derived exosomes isolated using the 500 kDa filter are referred to as "500 kDa exosomes".

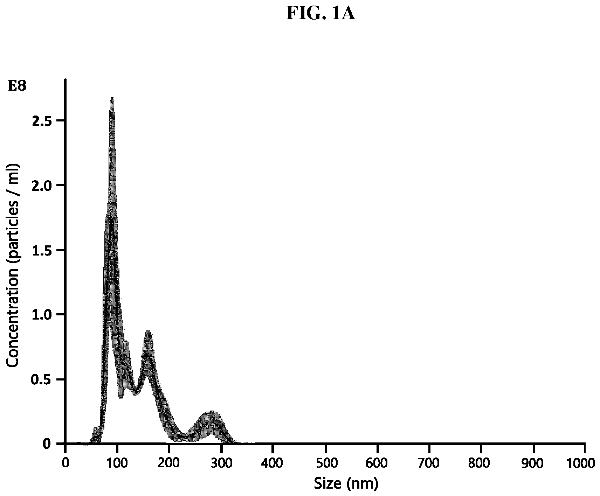

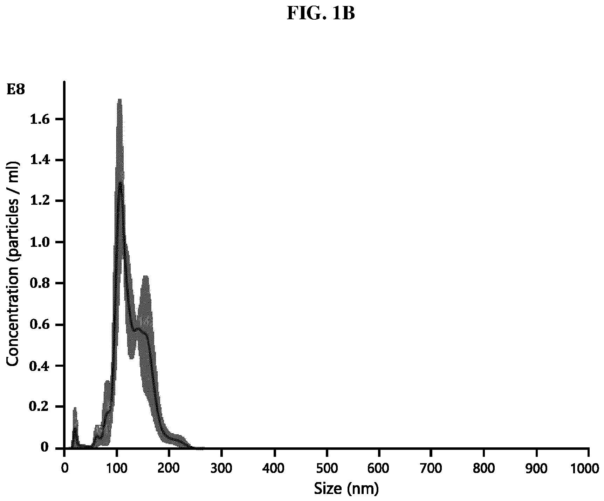

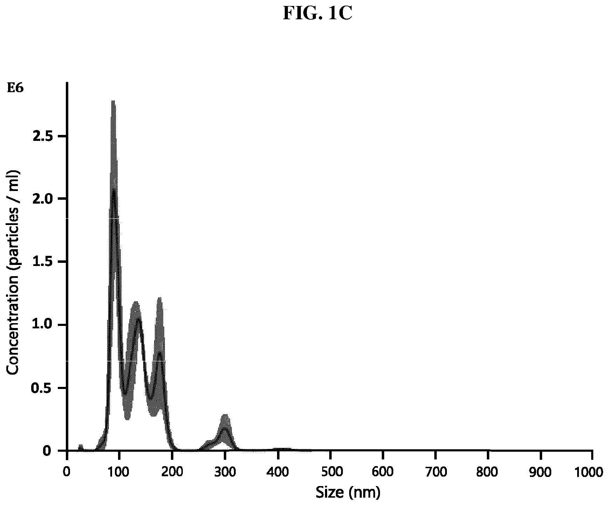

The size and concentration of the isolated rose stem cell-derived exosomes were analyzed by nanoparticle tracking analysis (NTA) using NS300 (purchased from Malvern Panalytical) (FIG. 1A and FIG. 1B).

### Example 4: Melanogenesis inhibitory effect

The whitening effect of the conditioned media of rose stem cells or the rose stem cell-derived exosomes (each of 100 kDa exosomes and 500 kDa exosomes) prepared in Example 3 was evaluated through the degree of inhibition of melanogenesis in mouse melanoma cells. The melanoma cells are cells derived from mouse melanoma and are cells that secrete a black pigment called melanin. Melanoma cells were seeded into a 48-well plate at 7,000 cells per unit area and cultured for 24 hours under 5% CO₂ and 37°C.

The conditioned media of rose stem cells were diluted to 20% in a culture medium mixed with α-MSH, a melanin synthesis stimulant, melanoma cells were treated with the diluted conditioned media, and then cultured for 48 hours under 5% CO₂ and 37°C. In addition, the rose stem cell-derived exosomes (100 kDa exosomes or 500 kDa exosomes) were diluted to 20% and 6% in a culture medium mixed with α-MSH, melanoma cells were treated with the diluted exosomes, and then cultured for 48 hours under 5% CO₂ and 37°C. At this time, the treatment concentration of the rose stem cell-derived exosomes (100 kDa exosomes or 500 kDa exosomes) was made to be 2.5×10⁹ particles/mL (20%) and 8.0×10⁸ particles/mL (6%). Arbutin (final concentration: 1 mM), a positive control, was also diluted in a culture medium mixed with α-MSH, and melanoma cells were treated with the diluted arbutin. Thereafter, the culture medium was recovered, and the melanoma cells were washed using a washing solution (PBS; purchased from ThermoFisher).

The recovered culture medium was mixed with CCK-8 assay reagent (Dojindo), the mixture was incubated for 1 hour and 30 minutes under 5% CO₂ and 37°C, and then the supernatant of the mixture was transferred to a 96-well plate to measure the absorbance at 405 nm. In addition, after washing the melanoma cells in the 48-well plate with the washing solution, the washed melanoma cells were treated with 1N NaOH mixed with 10% DMSO (purchased from MerckMillipore), and then the plate was sealed and heated for 30 minutes at 80°C to extract melanin from the melanoma cells. The amount of extracted melanin was calculated by measuring the absorbance at 405 nm.

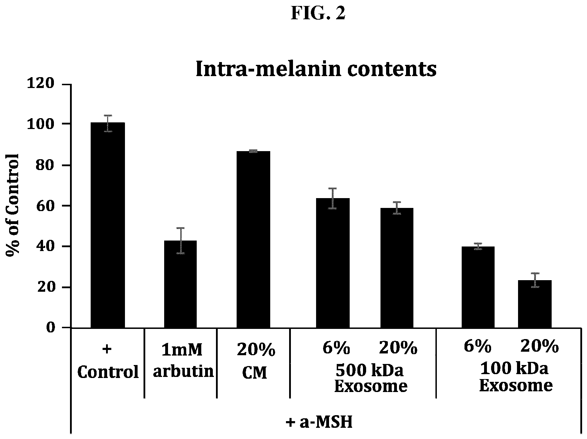

As a result, it was confirmed that the rose stem cell-derived exosomes according to the present invention (100 kDa exosomes and 500 kDa exosomes) decreased the melanin synthesis in melanoma cells in a concentration-dependent manner, and in particular, remarkably decreased the melanin synthesis in melanoma cells as compared with the conditioned media of rose stem cells (FIG. 2). In addition, it was confirmed that the effect for decreasing melanin synthesis (whitening effect) of 100 kDa exosomes was equivalent to arbutin which is widely used as a whitening agent. Thus, the composition comprising the rose stem cell-derived exosomes of the present invention as an active ingredient has a skin whitening effect and is useful as an active ingredient of a cosmetic composition for whitening a skin.

### Example 5: Evaluation of effect on cell viability

In this example, in order to evaluate the toxicity of the exosomes in B16F10 cells, which are mouse melanoma cells, B16F10 cells were treated with the conditioned media of rose stem cells or the rose stem cell-derived exosomes (100 kDa exosomes or 500 kDa exosomes) prepared in Example 3 and then the proliferation rate of the cells was confirmed.

After B16F10 cells were suspended in DMEM containing 10% FBS, the cells were seeded into a 48-well plate at 7,000 cells per well and cultured in an incubator for 24 hours under 5% CO₂ and 37°C. After 24 hours, the culture medium was removed, and B16F10 cells were treated with each of arbutin as a positive control, the conditioned media of rose stem cells, the 100 kDa exosomes and the 500 kDa exosomes at the same concentrations as in Example 4 and cultured for 48 hours to evaluate the cell viability. The cell viability was measured using CCK-8 assay reagent (purchased from Dojindo) and a microplate reader (purchased from Molecular Devices).

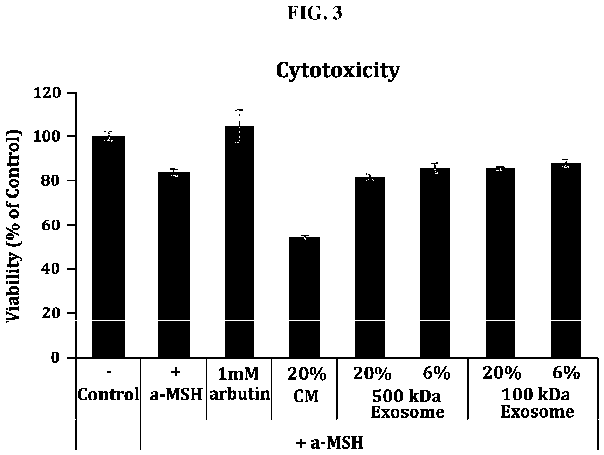

As a result of measuring the cell viability, it was confirmed that the cell viability decreased when the cells were treated with the filtrates of conditioned media of rose stem cells, whereas there was no change in the cell viability when the cells were treated with the rose stem cell-derived exosomes (100 kDa exosomes and 500 kDa exosomes) of the present invention. Thus, the cell viability is less affected by the rose stem cell-derived exosomes, and the rose stem cell-derived exosomes are less cytotoxic (FIG. 3).

Although the present invention has been described with reference to the embodiments, the scope of the present invention is not limited to these embodiments. Any person skilled in the art will appreciate that various modifications and changes are possible without departing from the spirit and scope of the present invention and these modifications and changes also fall within the scope of the present invention.

## Claims

1. A cosmetic composition for whitening a skin comprising exosomes derived from rose stem cells as an active ingredient.

2. The cosmetic composition of claim 1, wherein the cosmetic composition is a cream or a lotion.

3. The cosmetic composition of claim 1 or 2, wherein the rose stem cells are obtained by inducing a callus from rose embryos or leaves and then culturing cells of the callus.

4. A cosmetic method for whitening a skin comprising, applying a cosmetic composition for whitening a skin comprising rose stem cell-derived exosomes as an active ingredient to a mammalian skin.

5. The cosmetic method of claim 4, wherein the method comprising steps of : (a) applying the cosmetic composition directly to the mammalian skin; or (b) contacting or attaching a patch, a mask pack or a mask sheet, which has the cosmetic composition applied thereto or soaked therein, to the mammalian skin; or sequentially performing (a) and (b).

6. The cosmetic method of claim 5, wherein the cosmetic composition is a lotion or a cream in step (a).

7. The cosmetic method of claim 5 or 6, further comprising step (c) removing the patch, the mask pack or the mask sheet from the mammalian skin after step (b), and applying the cosmetic composition to the mammalian skin.

8. The cosmetic method of claim 7, wherein the cosmetic composition is a lotion or a cream in step (c).

9. The cosmetic method of any one of claims 4 to 6, wherein the mammal is at least one selected from the group consisting of a human, a dog, a cat, a rodent, a horse, a cattle, a monkey and a pig.
